# EUROPEAN PATENT APPLICATION

(11) **EP 1 006 185 A1**
(43) Date of publication of application: **07.06.2000**
(21) Application number: 98203372.2
(22) Date of filing: 06.10.1998
(51) Int. Cl.: C12N 15/12, C12N 5/10, C07K 14/47, C07K 14/82, C07K 16/32, A61K 38/17, A61K 39/395, A61K 31/70, A01K 67/027, G01N 33/50, C12Q 1/68

(54) **Tissue-specific splice variant of recombination-repair gene rad51b associated with t(12;14)-uterine leiomyomas**

(71) Applicant: Vlaams Interuniversitair Instituut voor Biotechnologie vzw., 9052 Zwijnaarde (BE)
(72) Inventor: Van de Ven, Willem Jan Marie, 3000 Leuven (BE); Schoenmakers, Eric, 5663 ST Geldrop (NL)

(57) **Abstract**

The current invention concerns new splice variants of RAD51B involved in the formation of uterine leiomyomas and the use of the products encoded by these variants for, among others, the development of therapeutic protocols and/or drugs preventing or treating uterine leiomyoma.

SEQUENCE LISTING

(1) GENERAL INFORMATION:

(i) APPLICANT:
(A) NAME: Vlaams Interuniversitair Instituut voor
Biotechnologie VZW
(B) STREET: Rijvisschestraat 120
(C) CITY: ZWIJNAARDE
(E) COUNTRY: Belgium
(F) POSTAL CODE (ZIP): B - 9052
(G) TELEPHONE: 32 9 244 66 11
(H) TELEFAX: 32 9 244 66 10

(ii) TITLE OF INVENTION: Tissue-specific novel splice variants of
recombination-repair gene RAD51B associated with
t(12;14)-uterine leiomyomas.

(iii) NUMBER OF SEQUENCES: 14

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98.203.372.2

(2) INFORMATION FOR SEQ ID NO: 1:

(i) SEQUENCE CHARACTERISTICS:
(A) LENGTH: 1011 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 1:

GTTGTTAGAA CTGTGGCCAG AGTCAGCCCC AGGTCAGAAT GCTGGTCAGC AGCAGACTCA 60

CATCCCAGCC CTACCTTCCT GATACCTGAT GTGGTGCTCA GAAAAACAAG GAATCCCAGG 120

CTGTTACCCA GTGTCTCCAT GTGCCCTCCA CAGATGCACT CAACTGTCCT CTCTTCTGCT 180

CTTCCTCCTA CAAACTGGGC AACTGGAATC AGAACAGAGA CTTTGGATGA GCATCCAGTG 240

TAACCCAGCC TAGTTTTTCA CCACGACTTC CAGATCTACA TTTAACATCC GACAGCAACT 300

ATAATTCTAC TTTCTGAACC TCCAGGGAAG TTGCTTCCTT GTCCCTTTCA GCAAAGGATG 360

TCATGGTTTT ACAATGTCTC CAGTGAAAAA GTTCTTCTTC TCTACATCTT CCTCCCACTC 420

CTACAACCCT TGATCCCCAC GTGAGATCTC ATTTCCTCCA GCTGGGTCTT CTGCAGAGTG 480

ATTAGAGCTT CTGCCCTCCT TGCAGCATCT CTATTGTAAA AGGATTGCTG AAGCTTATTA 540

AGCCTTGCAG CTCCTAGGAG GAAAGGTACC ATATAGAAAT AAAACAAATC AACAAATTAG 600

TGTGTTTTGT AGAAGTTGAA TAGGACTCCA CCTATCTGCA TGAGTGTGCA CACTGTGCAG 660

TCGCACAGGC AGTCTCAAAT GGGAATTTGG AAAAAGCTAA GCTTTTTCAC TTGAGAAAGG 720

TTTACATATT CTTTGCACCT GGGCACACAC AGCACAGGCT GTTCTCAGGA CTCCCACCAG 780

ATTAGGCCTG GGACATGACA GAGCCTGTCA GGTCCACGTT TGCTTTCCAG TCTCCTTGCT 840

TCCAGTCTAG CTGTGGCCTG ACTCCAAGTC CAGTGTAAGA TTTTTTTTTT TCAGCAATAA 900

GGAATCGGTT CATTCACCTG TACGAAAACC AGTGGGAGGA TCATGCATAA GGCTCATATC 960

CTTGCTGTGA ATTTCAAAGG ACTGGCTAAG TAAATAAAAT TATCATGCCA A 1011

(2) INFORMATION FOR SEQ ID NO: 2:

(i) SEQUENCE CHARACTERISTICS:
(A) LENGTH: 80 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 2:

Val Val Arg Thr Val Ala Arg Val Ser Pro Arg Ser Glu Cys Trp Ser
1               5                   10                  15

Ala Ala Asp Ser His Pro Ser Pro Thr Phe Leu Ile Pro Asp Val Val
            20                  25                  30

Leu Arg Lys Thr Arg Asn Pro Arg Leu Leu Pro Ser Val Ser Met Cys
        35                  40                  45

Pro Pro Gln Met His Ser Thr Val Leu Ser Ser Ala Leu Pro Pro Thr
    50                  55                  60

Asn Trp Ala Thr Gly Ile Arg Thr Glu Thr Leu Asp Glu His Pro Val
65                  70                  75                  80

(2) INFORMATION FOR SEQ ID NO: 3:

(i) SEQUENCE CHARACTERISTICS:
(A) LENGTH: 34 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "adapter"

(xi) SEQUENCE DESCRIPTION: SEQ ID NO: 3:

AAGGATCCGT CGACATCTTT TTTTTTTTTT TTTT 34

(2) INFORMATION FOR SEQ ID NO: 4:

(i) SEQUENCE CHARACTERISTICS:
(A) LENGTH: 18 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..18

(xi) SEQUENCE DESCRIPTION: SEQ ID NO: 4:

TACAGCAGTT TTTCACTA 18

(2) INFORMATION FOR SEQ ID NO: 5:

(i) SEQUENCE CHARACTERISTICS:
(A) LENGTH: 18 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..18

(xi) SEQUENCE DESCRIPTION: SEQ ID NO: 5:

CTTCAGCCCA GGGACAAC 18

(2) INFORMATION FOR SEQ ID NO: 6:

(i) SEQUENCE CHARACTERISTICS:
(A) LENGTH: 18 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..18

(xi) SEQUENCE DESCRIPTION: SEQ ID NO: 6:

TGAAGAACCA GGCCTTCC 18

(2) INFORMATION FOR SEQ ID NO: 7:

(i) SEQUENCE CHARACTERISTICS:
(A) LENGTH: 30 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..30

(xi) SEQUENCE DESCRIPTION: SEQ ID NO: 7:

CAUCAUCAUC AUCGCCTCAG AAGAGAGGAC 30

(2) INFORMATION FOR SEQ ID NO: 8:

(i) SEQUENCE CHARACTERISTICS:
(A) LENGTH: 30 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..30

(xi) SEQUENCE DESCRIPTION: SEQ ID NO: 8:

CUACUACUAC UAAGGGGACT TGGCAATAAG 30

(2) INFORMATION FOR SEQ ID NO: 9:

(i) SEQUENCE CHARACTERISTICS:
(A) LENGTH: 29 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..29

(xi) SEQUENCE DESCRIPTION: SEQ ID NO: 9:

GGGTAGCAAG AAACTAAAAC GAGTGGGTT 29

(2) INFORMATION FOR SEQ ID NO: 10:

(i) SEQUENCE CHARACTERISTICS:
(A) LENGTH: 28 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..28

(xi) SEQUENCE DESCRIPTION: SEQ ID NO: 10:

AAAAGATCCA ACTGCTGCTG AGGTAGAA 28

(2) INFORMATION FOR SEQ ID NO: 11:

(i) SEQUENCE CHARACTERISTICS:
(A) LENGTH: 29 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..29

(xi) SEQUENCE DESCRIPTION: SEQ ID NO: 11:

CUACUACUAC UAAAGGATCC GTCGACATC 29

(2) INFORMATION FOR SEQ ID NO: 12:

(i) SEQUENCE CHARACTERISTICS:
(A) LENGTH: 36 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..36

(xi) SEQUENCE DESCRIPTION: SEQ ID NO: 12:

CAUCAUCAUC AUTCACAAGA GCTGTGTGAC CGTCTG 36

(2) INFORMATION FOR SEQ ID NO: 13:

(i) SEQUENCE CHARACTERISTICS:
(A) LENGTH: 36 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..36

(xi) SEQUENCE DESCRIPTION: SEQ ID NO: 13:

CUACUACUAC UAGTCCTCTT CGGCAGACTC TTGTGA 36

(2) INFORMATION FOR SEQ ID NO: 14:

(i) SEQUENCE CHARACTERISTICS:
(A) LENGTH: 29 base pairs
(B) TYPE: nucleic acid
(C) STRANDEDNESS: double
(D) TOPOLOGY: linear

(ii) MOLECULE TYPE: other nucleic acid
(A) DESCRIPTION: /desc = "synthetic DNA"

(ix) FEATURE:
(A) NAME/KEY: primer_bind
(B) LOCATION:1..29

(xi) SEQUENCE DESCRIPTION: SEQ ID NO: 14:

CUACUACUAC UAAAGGATCC GTCGACATC 29

## Description

The present invention concerns new splice variants of RAD51B involved in the formation of uterine leiomyomas and the use of the products encoded by these variants for, among others, the development of therapeutic protocols and/or drugs preventing or treating uterine leiomyoma. Uterine leiomyoma is a common benign smooth muscle tumor occurring in 20-30% of women over 30 years of age leading to over 200,000 hysterectomies annually in the USA alone (Carlson, K.J. *et al. N. Eng. J*. *Med*. 28, 856-860 (1993). Although most patients with these steroid-dependent tumors are asymptomatic, symptomatic leiomyomas can be associated with abnormal uterine bleeding, pelvic pain, urinary dysfunction, spontaneous abortions, premature delivery or even infertility. These symptoms, combined with the high incidence of this benign smooth muscle tumor constitutes a major public health problem. Cytogenetic analysis of these tumors has revealed that over 60% of them have an apparently normal karyotype but also that they sometimes carry recurrent chromosome aberrations, for instance aberrations involving the short arm of chromosome 6 or the long arm of chromosome 12. Precise mapping of chromosome breakpoints of such recurrent chromosome translocations and molecular characterization of DNA regions immediately flanking such breakpoints has proven to be a successful approach to identify and isolate genes involved in tumor development.
Recently, application of such a research strategy on translocations in uterine leiomyoma involving either chromosome 12q13-15 or 6p21 has led to the finding that two members of the high mobility group (HMG) protein gene family, *HMGIC* and *HMGIY*, are frequently rearranged in such tumors. The developmentally regulated *HMGIC* and *HMGIY* genes encode closely related low molecular weight proteins, which are currently assumed to function as architectural factors in the nuclear scaffold and to be critical in the assembly of stereospecific transcriptional complexes. The frequent rearrangement of the *HMGIC* and *HMGIY* genes in uterine leiomyomas suggests that these genes are directly involved in the aberrant growth control observed in these tumors. Studies of a number of other benign solid tumors, - most of them in tissues of mesenchymal origin -, indicated that involvement of *HMGIC* and/or *HMGIY* is not restricted to uterine leiomyomas, suggesting that benign solid tumor formation might have a common genetic denominator. The possible role of HMG genes in growth control is further supported by results from gene targeting experiments in mice, which indicate that HMGIC plays an important role in mammalian growth and development, as inactivation of the *HMGIC* gene in mice resulted in the pygmy phenotype.

### Background of the invention

The evolution from classical genetics to molecular genetics and genetic engineering has revealed the immense potential of gene manipulation for basic science and medicine. The profound impact of genetics on modern society is becoming increasingly evident in many different areas of our daily life. In health care, identification and characterization of genes involved in genetic diseases, such as cancer, has already led to significant contributions to diagnosis and to both an understanding of therapy and suggestions for novel therapies (including gene therapy). Molecular cytogenetic research on recurrent chromosome aberrations in uterine leiomyoma as well as other benign solid tumors has recently led to the finding of the novel multi-tumor aberrant growth (MAG) gene, *HMGIC*; the first known benign solid tumor gene (Schoenmakers *et al*., 1995, Nature Genetics 10:436-444). The link between *HMGIC* and the development of uterine leiomyomata provides the first insight in the genetic basis of this disease and may provide new opportunities to those working in the field of female health care to develop alternative therapeutic approaches. The implication of the *HMGIC* gene in uterine leiomyoma was discovered by applying a variety of technologies of modem molecular genetics.
Starting with cytogenetic analysis of recurrent chromosome aberrations, it became clear that uterine leiomyomas are often characterized by a t(12;14)(q15;q23-24) translocation. After having established a number of cell lines from primary leiomyomas of the uterus, the chromosome 12q breakpoints could be assigned to a particular region of chromosome 12 by *in situ* hybridization techniques. Subsequently, this region was defined molecularly by isolating the YAC (yeast artificial chromosome) clones containing human DNA fragments representing this part of human chromosome 12. On the basis of these data, the chromosome 12 breakpoint cluster region could be defined very accurately and subsequent gene hunting experiments in the breakpoint cluster region led to the finding that the high mobility group protein gene, *HMGIC*, is consistently affected by the chromosomal rearrangements. The *HMGIC* gene encodes a nuclear protein which is assumed to act as an architectural transcription factor.
In contrast to the highly variable translocation partners in most mesenchymal tumors, uterine leiomyomas almost exclusively utilize chromosome 14q23-24 as partner of 12q15 (Mitelman, F. Catalog of chromosome aberrations in cancer. 4th Edition, 1994. Wiley-Liss, New York).
Leiomyomas are the most frequent uterine tumors and they occur, as mentioned above, in about 20-30 % of all women over 30 years. They are benign mesenchymal solid tumors which sometimes remain asymptomatically for a relatively long period of time. Cytogenetic analysis of over 800 uterine leiomyomas has indicated that more than 65% of these tumors possess a normal female karyotype and that about 35% have clonal chromosomal aberrations (Mitelman, Catalog of chromosome aberrations in cancer. 4th Edition, 1994. Wiley-Liss, New York). It is possible to distinguish a number of cytogenetically abnormal subgroups: tumors with changes involving the short arm of chromosome 6 (6p21), tumors with deletions in the long arm of chromosome 7 (presumably del(7)(q21.2-q31.2)), tumors with trisomy 12, and tumors with chromosome 12q14-15 changes with most of the chromosomes acting as possible translocation partner, although often t(12;14)(q15;q23-24) is observed. In the remaining cytogenetically abnormal uterine leiomyomas, various non-recurrent anomalies have been found. Chromosomal translocations involving the region 12q13-15 are observed in a variety of other solid tumors as well (Mitelman, 1994). Well known examples are lipomas and pleomorphic adenomas of the salivary glands. Furthermore, the involvement of chromosome 12q13-15 has been reported for subgroups of among others rhabdomyosarcomas, hemangiopericytomas, chondromas, harmatomas of the lung, and for tumors of the breast. The frequent rearrangements of the region 12q13-15 in solid tumors raises questions about the number and function of putative cancer gene(s) assumed to be located in this region.

### Detailed description of the invention

Currently, leiomyomas are treated either by surgery or endocrine therapy (i.e. the administartion of anti-estrogens). Whereas application of the former therapy contributes significantly to the high costs of public health, the latter approach has been demonstrated in most cases to only induce a temporary regression of tumor size. The approach of isolation and subsequent structural and functional characterization of the gene(s) targeted by the (major) chromosomal aberrations will certainly provide insight at the basis of the process of tumorigenesis in this tumor type. These new insights into the formation of uterine leiomyomata might also provide new therapeutic strategies. The subsequent development of an animal model system, for instance a mouse model system, for uterine leiomyomas might prove to be a major tool for the development of new therapeutic strategies.

It is our finding that the recombinational repair gene *RAD51B* on chromosome 14q23-24 is the preferential translocation partner of *HMGIC* in uterine leiomyomas. In the context of developing leiomyomas, the pathogenetically critical sequences seem to reside in the last coding exon of novel *RAD51B* isoforms, which encode a domain containing a putative transmembrane anchor and are expressed in the uterus but apparently not in a wide variety of other tissues tested. By FISH, 3'-RACE, and RT-PCR analysis, consistent chromosomal rearrangements within *RAD51B* have been demonstrated and expression of fusion transcripts, structurally resulting in an allelic knock-out of the uterine isoform of *RAD51B* and confirming a pleiotropic function of this gene.

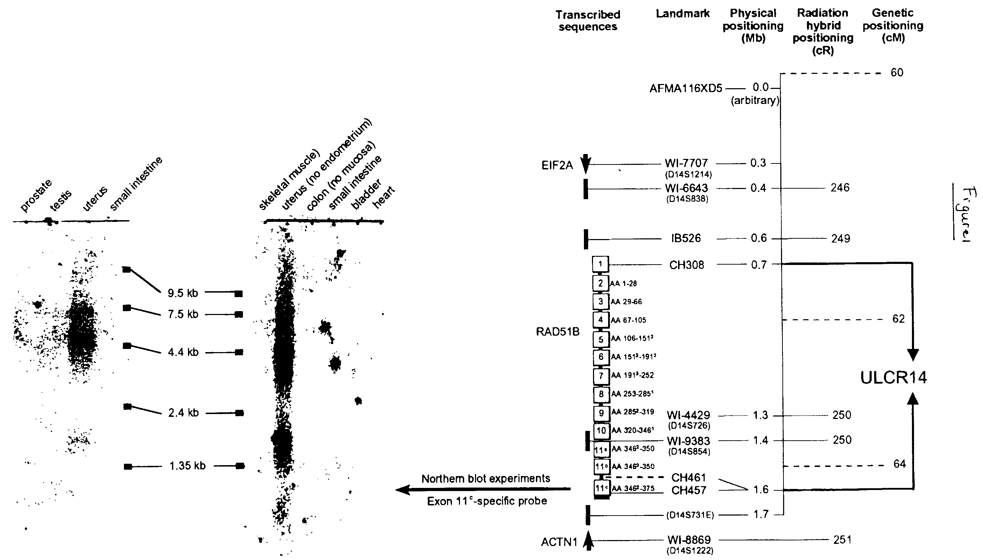

It is our invention that a specific nucleotide sequence, indicated in this description as SEQ.ID.NO.1, surprisingly is a novel hitherto unknown splice variant of *RAD51B* (Albala, J.S. *et al. Genomics* , 1997,46, 476-479) {also known as *R51H2*; Cartwright, *et al. Nucleic Acids Res*. 26, 1653-1659 (1998) and *hREC*2; Rice, M.C. *et al*. *Proc*. *Natl*. *Acad*. *Sci*. *USA* 94, 7417-7422 (1997) and member of the *recA*/*RAD51* recombination-repair gene family; Shinohara, A. *et al*. *Cell* 69, 457-470 (1992)} being the chromosome 14 target gene in uterine leiomyomas with t(12:14), which leads to its dysregulation and suggests a pleiotropic role of this gene.
Hereto a YAC contig (2.7 Mb) encompassing the chromosome 14 breakpoint cluster region (ULCR14) was constructed. Said ULCR14 was mapped by FISH (fluorescence in situ hybridization) analysis to a genomic interval of about 900 kb (Fig. 1). Among transcribed sequences residing within ULCR14 (Fig. 1) (uterine leiomyoma breakpoint region of chromosome 14), those detected by Northern blot analysis with probe CH457 were particularly intriguing, since they appeared to be part of transcripts of approximately 4.4 kb (major band), 7.0 kb and 1.6 kb (minor bands) expressed at low levels in uterus but not in any of the many other tissues and cell lines tested (Fig. 1). Evaluation of related cDNAs revealed novel sequences fused to sequences of the *RAD51B* gene. However *RAD51B* expression (reported as 1.8 kb mRNA) is ubiquitous and abundant, and, consistent with its proposed role in DNA repair, radiation-inducible and most prominent in tissues active in recombination. This clearly indicates the existence of as yet unknown, uterine splice variants of *RAD51B*.

Therefore the invention concerns a DNA sequence partially encoding a protein of which the corresponding mRNA is almost completely restricted to expression in the uterus ; said sequence maps on human chromosome 14q23-24 or encoding an immunologically active and/or functional fragment of such a protein, selected from the group consisting of:
(a) DNA sequences comprising a nucleotide sequence encoding a protein comprising the amino acid sequence as given in SEQ ID NO: 2
(b) DNA sequences comprising a nucleotide sequence as given in SEQ ID NO: 1;
(c) nucleotide sequences hybridizing with one or both of the strands of a DNA sequence as defined in (a) or (b) and encoding an amino acid sequence which is at least 80% identical to the amino acid sequence encoded by the DNA sequence of (a) or (b);
(d) DNA sequences, the nucleotide sequence of which is degenerated as a result of the genetic code to a nucleotide sequence of a DNA sequence as defined in any one of (a) to (c); and
(e) DNA sequences encoding a fragment of a protein encoded by a DNA sequence of any one of (a) to (d).

Part of the current invention is the amino acid sequence derivable from SEQ.ID.NO.1 and depicted as SEQ.ID.NO.2. It should be noted that the first nucleotide of SEQ.ID.NO.1, viz. "G", is coded by exon 10 making up (with the first two "T"-residues of exon 11c) a triplet "GTT" coding for V(aline). Consequently in SEQ.ID.NO.2 the first amino acid given "V" (valine) is made up from "G" originating from exon 10 and "TT" originating from exon 11c.
As a consequence of this intra-codon splicing it can be postulated that exons 9 and 10 can be skipped in the process. The last "G" nucleotide present in exon 8 is found coupled to the start of exon 11c ("TT") making up again a triplet "GTT" coding for V(aline).
For sake of clarity table 1 is herewith provided wherein the intron/exon junction sequences of human *RAD51B* gene are depicted.

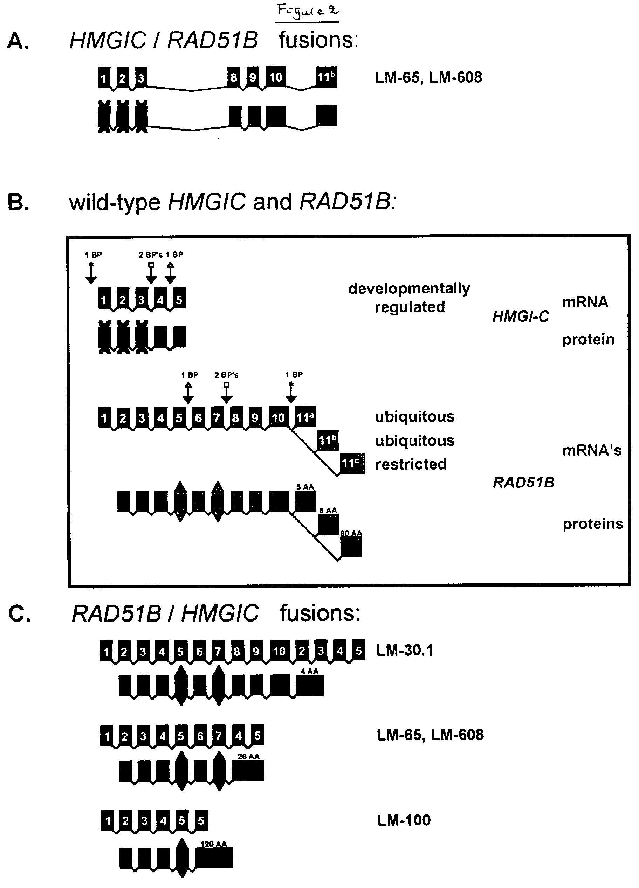

The established exon/intron distribution revealed that *RAD51B* almost completely encompasses ULCR14 (Fig. 1), with all breakpoints upstream of alternative terminal exon 11c. Exon 11c provides the related RAD51B splice variants with a unique stretch of 80 amino acids containing a putative transmembrane segment. The so-called 3'-RACE and RT-PCR analysis was used (Fig. 2) and the t(12;14)-induced hybrid transcripts in uterine leiomyomas were evaluated revealing that the reading frames of the *RAD51B*/*HMGIC* transcripts code for variant truncations of the RAD51B protein, all lacking the 80 amino acid domain and containing variable carboxy-terminal ends, encoded by frameshifted *HMGIC* sequences. The largest subgroup of *RAD51B*/*HMGIC* hybrids is expected to resemble the fusion transcript as found in LM-30.1/SV40, because the chromosome 12 breakpoints have been reported to frequently map upstream of *HMGIC* (Schoenberg, F.M. *et al. Genes Chromosom*. *Cancer* 17, 1-6 (1996)), excluding also the reciprocal *HMGIC*/*RAD51B* form. Anyhow, such reciprocal fusion transcripts were detected only in a few cases (Fig. 2), reducing the chances of a pathogenetically critical role dramatically. Expression in the tumors of wild-type *RAD51B* and *HMGIC* transcripts was demonstrated by RT-PCR.

According to the current invention allelic knock-out of particular uterine splice variants of *RAD51B* result in expression of truncated and carboxy-terminally altered RAD51B proteins. This finding together with dysregulation of *HMGIC* appear to be tumor-specific features of uterine leiomyomas with t(12;14)(q15;q23-24) translocations. Pathogenetically, the observed dysregulations of *RAD51B* and *HMGIC* most likely act synergistically, the former possibly conveying a tissue-dependent and the latter a more common effect. Furthermore, the link of *RAD51B* to tumorigenesis suggests that this gene may play a role in addition to its presumed recombination-repair function. In line with such a pleiotropic, possibly splicing-dependent, role of RAD51B are observations with highly related family member RAD51A, which has been shown to promote ATP-dependent homologous pairing and strand transfer reactions *in vitro* (Bauman, P. *et al*. *Cell* 87, 757-766 (1996)) to play an essential role in mammalian cell viability (Lim, D.S. *et al*. *Mol*. *Cell. Biol*. 16, 7133-7143 (1996)) and to be linked etiologically to cancer, because of its interaction with p53 (Stürzbecher, H.W. *et al*. *EMBO J.* 15, 1992-2002 (1996)), BCRA1 (Scully, R. *et al*. *Cell* 88, 265-275 (1997)), and BCRA2 (Sharan, S.K. *et al*. *Nature* 386, 804-810 (1997)). Our invention offers new viewpoints and unique tools for dissecting the tumorigenic processes in uterine leiomyoma and for deciphering functions of the intriguing recombination-repair gene family.

To the scope of the present invention also belongs a nucleic acid molecule of at least 15 nucleotides in length designed to hybridize, preferably specifically, with a DNA sequence of the invention or with a complementary strand thereof.

In addition a vector comprising a DNA sequence according to the invention and said vector which can be an expression vector wherein said DNA sequence is operably linked to one or more control sequences allowing the expression in host cells belong to the scope of the current invention as well.
Furthermore the invention relates to a method for the production of an uterus specific protein or an immunologically active or functional fragment thereof comprising culturing a host cell under conditions allowing the expression of the protein and recovering the produced protein from the culture.
An uterus specific protein or an immunologically active or functional fragment thereof encodable by said DNA sequence, an antibody or antibodies or antibody mixture specifically recognizing the protein or a fragment or epitope thereof are part of the invention.
Another aspect of the invention is a method for identifying and obtaining an activator or inhibitor of one or more uterus specific proteins comprising the steps of:
(a) combining a compound to be screened with a reaction mixture containing the protein of the invention and a system capable of interacting with the protein under suitable conditions;
(b) maintaining said reaction mixture in the presence of the compound or a sample comprising a plurality of compounds under conditions which permit interaction of the protein with said system;
(c) identifying or verifying a sample and compound, respectively, which leads to suppression or activation of said system.
Said compound obtained or identified by the method mentioned can be an activator or inhibitor of uterus specific proteins.
A diagnostic composition comprising a nucleotide sequence of the invention, the earlier mentioned vector, the protein of the invention, and/or an antibody directed thereto are part of the invention too.
The nucleotide sequence and the derivatives thereof mentioned above are used for the preparation of a medicament to treat patients suffering from a or multiple uterine leiomyoma.

The finding of the role of high mobility group protein genes and especially the novel RAD51B splice variant according to the current invention in the formation of uterine leiomyoma is the basis for research aiming at the development of alternative therapeutic protocols for this common disease. One possible approach involves genetic engineering to create an animal model, such as a mouse model, for uterine leiomyoma. On the one hand, such a model is useful in further dissection of the molecular mechanisms involved in the neoplastic transformation of uterine smooth muscle to uterine leiomyoma. On the other hand, it can be used later on in testing therapeutic strategies, e.g. screening of candidate drugs.

For sake of sufficiency some protocols used in the current invention are given below.

### Cell culture.

Cell lines containing the classical t(12;14), which were used in this study were established through transfection of primary leiomyoma tumor cells with a construct containing the SV40 early region. Cells were grown in DMEM/F12 supplemented with 10% fetal bovine serum (GIBCO/BRL) and were assayed by standard cytogenetic techniques at regular intervals.

### Libraries.

YAC clones were isolated from the CEPH mark 1 and mark 3 YAC libraries. Local copies of these libraries were screened using a PCR-based strategy in initial rounds of screening, followed by colony hybridization analysis in the final round of screening. BAC clones were identified on commercially obtained high density grids (Research Genetics Inc.) by colony hybridization.

### DNA preparation.

Agarose plugs containing high-molecular weight yeast and YAC DNA (equivalent to 1 x 10⁹ cells/ml) were prepared as described before and stored at room temperature in lysis buffer (1% lithium dodecyl sulphate, 100 mM EDTA, 10 mM Tris-HCl pH 8.0). Plugs were thoroughly dialysed against four changes of 25 ml T₁₀E₁ (10 mM TRIS-HCl, 1 mM EDTA, pH 8.0) followed by 2 changes of 0.5 ml 1 x restriction buffer before they were subjected to either restriction enzyme analysis or YAC-end rescue. BAC DNA was isolated using a slightly modified cosmid prep protocol.

### YAC and BAC analysis.

After restriction enzyme digestion, YAC plugs or "liquid" BAC DNA along with appropriate molecular weight markers were loaded onto 1% N.A.agarose (Pharmacia) / 0.25 x TBE gels, sealed with LMP-agarose (in the case of YAC plugs) and size fractionated using a Contour-Clamped Homogeneous Electric Field (CHEF) apparatus (Biorad). Runs were for 18 h at 6.0 V/cm using a pulse angle of 120 degrees and pulse times appropriate to the resolution required. Electrophoresis was performed at 14 °C in 0.25 x TBE. After electrophoresis, gels were stained, photographed, and UV crosslinked using a stratalinker (Stratagene) set at 120 mJ. DNA was subsequently blotted onto Hybond N+ membranes (Amersham Pharmacia Biotech) for 4-16 h using 0.4 N NaOH as transfer buffer. Filters were hybridized and washed according to standard procedures. Kodak XAR-5 films were exposed at -80 °C for 3-16 h, depending on probe performance. Intensifying screens (Kyokko special 500) were used.

### Generation of YAC- and BAC-derived STSs.

Sequence data from YAC insert ends were obtained using a vectorette-PCR procedure in combination with direct DNA sequencing analysis. Sequence data from BAC insert ends were obtained by direct cycle sequencing using either standard FITC-labeled or Energy Transfer (C-FAM-labelled) T7 and SP6 primers (Amersham Pharmacia Biotech). After sequence analysis, primer pairs were developed using the OLIGO computer algorithm.

### DNA labeling.

DNA from YACs, BACs, cosmids, PCR products and oligonucleotides was labeled using a variety of techniques. For fluorescence in situ hybridization, cosmid or BAC clones or inter-Alu PCR products of YACs were biotinylated with biotin-14-dATP or digoxigenin-11-dUTP (Boehringer) by nick translation. For filter hybridizations probes were radio-labeled with 32P-dCTP using random hexamers. In case of PCR-products smaller than 200 bp in size, a similar protocol was applied, but specific oligonucleotides were used to prime labeling reactions. Oligonucleotides were labeled using radioactive labeled ATP.

### Nucleotide sequence analysis.

Nucleotide sequences were determined according to the dideoxy chain termination method using the AutoRead Sequencing Kit or the Thermo Sequenase Cycle Sequencing System (Amersham Pharmacia Biotech). Sequencing results were obtained using an A.L.F. DNA sequencerTM (Pharmacia Biotech) on standard 30 cm, 6% Hydrolink^{R}, Long Range™ gels (AT Biochem). Results were evaluated using the Lasergene Biocomputing Software package (DNASTAR).

### PCR reactions.

PCR amplifications were carried out using a GeneAmp PCR system 2400 or 9600 or a DNA Thermal Cycler (Perkin Elmer) in final volumes of 50 µl containing 10 mM Tris-HCl pH 8.3, 50 mM KCl, 1.5 mM MgCl2, 0.01 % gelatine, 2mM dNTP's, 20 pmole of each oligonucleotide (Eurogentec), 2.5 units of Amplitaq (Perkin-Elmer Cetus), and 5 ng (for cosmids) to 100 ng (for YAC superpools) of template DNA. For GC-rich templates DMSO was added to a final concentration of 10%. After initial denaturation for 5 min at 94 °C, 30 amplification cycles were performed each consisting of denaturation for 10 sec at 94 °C, annealing for 20 sec at the appropriate temperature and extension for 30 sec (1 min at 72 °C). The PCR reaction was completed by a final extension at 72 °C for 5 min. Results were evaluated by analysis of 10 µl of the reaction product on polyacrylamide minigels.

### Rapid amplification of cDNA ends (RACE) and RT-PCR.

Rapid amplification of 3' cDNA-ends (3'-RACE) and RT-PCR was performed using part of a modified 3' Exon Trapping protocol. For first strand cDNA synthesis, non-specific adapter primer AP2: 5' AAG GAT CCG TCG ACA TC(T)17 3' or HMGI-C specific primer 95C3362: 5' TAC AGC AGT TTT TCA CTA 3' was used.

For the specific isolation of 5' sequences of HMGI-C fused to 3' ectopic sequences of RAD51B, the following primer pairs were applied: In the first PCR round the following specific "forward primer" (situated within exon 1 of HMGI-C) was used: 5' CTT CAG CCC AGG GAC AAC 3' in combination with the following "reverse primer" (situated within the last common exon of RAD51B): 5' TGA AGA ACC AGG CCT TCC 3'. In the second round the following tailed, specific "forward primer" (nested as compared to the one used in the first round) was used: 5' CAU CAU CAU CAU CGC CTC AGA AGA GAG GAC 3' (situated within exon 1 of HMGI-C) in combination with: 5' CUA CUA CUA CUA AGG GGA CTT GGC AAT AAG 3' (situated within the last common exon of RAD51B).

For isolation of possible reciprocal products (5' sequences of RAD51B fused to 3' sequences of HMGI-C), the following primer combinations were used: In the first PCR round the following specific "forward primer" (situated within the first coding exon of RAD51B) was used: GGG TAG CAA GAA ACT AAA ACG AGT GGG TT 3' in combination with one of the following "reverse primers: 5' AAA AGA TCC AAC TGC TGC TGA GGT AGA A 3' (situated within the last exon of HMGI-C) or UAP2: 5' CUA CUA CUA CUA AAG GAT CCG TCG ACA TC 3'. In the second round the following tailed, specific forward primer (nested as compared to the one used in the first round) was used: 5' CAU CAU CAU CAU TCA CAA GAG CTG TGT GAC CGT CTG 3' (situated within the first coding exon of RAD51B) in combination with either: 5' CUA CUA CUA CUA GTC CTC TTC GGC AGA CTC TTG TGA 3' (situated within the last exon of HMGI-C and nested as compared to the one used in the first round) or UAP2: 5' CUA CUA CUA CUA AAG GAT CCG TCG ACA TC 3'.

CAU-tailing of the second round primers allowed the use of the directional CloneAmp cloning system (GIBCO/BRL).

### Fluorescence in situ hybridization.

Cells of various tumor cell lines were arrested in metaphase using colcemid after which FISH analysis was performed essentially as described before. Inter-Alu-PCRed YAC DNA or BAC DNA was labelled with either biotin-14-dATP (Boehringer) or digoxigenin-11-dUTP (Boehringer) using respectively the Bionick labeling system (GIBCO/BRL) or the nick translation system (GIBCO/BRL). The identity of chromosomes was established by simultaneous DAPI-banding. Vectashield antifade medium (Vector Laboratories, Inc.), containing DAPI (0.5 µg/ml) was added 15 min before slides were analyzed on a Zeiss Axiophot fluorescence microscope equipped with a cooled CCD camera system (Photometrics) using Quips Smart Capture FISH imaging software (Vysis Inc.). Images were printed with a Phaser 440 sublimation primter (Tektronix).

In order to clarify and/or explain the invention some examples are provided herewith.

### EXAMPLES

### Development of a 14q23-24 YAC and BAC contig.

Using as starting points STS RM121 (corresponding to genomic, t(12;14) breakpoint flanking sequences residing on chromosome 14; Schoenmakers, H.F.P.M. *et al*. Identification, molecular cloning and characterization of the chromosome 12 breakpoint cluster region of uterine leiomyomas: in Genes Chromosom. Cancer. *11*: 106-118, 1994) and EST CH165 (corresponding to ectopic chromosome 14 sequences which were isolated following a *HMGI-C* 3' RACE experiment; Schoenmakers, E.F.P.M. The molecular basis of benign solid tumors: Discovery of a common genetic denominator on the long arm of human chromosome 12. Ph.D. Thesis, University of Leuven, Belgium, 1997.), a yeast artificial chromosome (YAC) and bacterial artificial chromosome (BAC) contig were constructed consisting numerous overlapping YACs and BACs as well as a corresponding rare-cutter physical map spanning about 2.7 Mb.

### FISH-definition of ULCR14.

Using well-characterized contig elements (mainly BACs or STS-specific genomic lambda clones) from this 2.7 Mb contig in FISH experiments, we were able to define a uterine leiomyoma breakpoint cluster region on chromosome 14q23-24 (ULCR14), in which all of the chromosome 14q23-24 breakpoints under investigation appeared to cluster. This cluster region, which by FISH analysis was defined as the region mapping between STS CH308 (contained within BAC 8A21) and EST CH457 (contained within BAG 547J11) comprised a genomic interval of about 900 kb (Fig. 1). FISH analysis on normal metaphase spreads confirmed the reported mapping of ULCR14 at the border between 14q23 and 14q24.
Subsequent integration (i.e. mapping) of published, well characterized landmarks situated ULCR14 at approximately 62-64 cM relative to the most proximal (14pter) polymorphic marker, at a radiation hybrid position of 249-250 cR, at 0.7-1.6 Mb distal to AFMA116XD5 (Fig. 1).

### Isolation and characterization of transcribed sequences within ULCR14.

Using a variety of gene-identification techniques (including random sequence sampling and 3' exon-trapping) several independent expressed sequences mapping within our 2.7 Mb contig were identified. Among transcribed sequences residing within ULCR14 (Fig. 1, right panel), those detected by Northern blot analysis with probe CH457 were particularly intriguing, since they appeared to be part of transcripts of approximately 4.4 kb (major band), 7.0 kb and 1.6 kb (minor bands) expressed at low levels in uterus but not in any of the many other tissues and cell lines tested (Fig. 1, left panel). Evaluation of corresponding cDNAs revealed CH457 sequences in-frame fused to sequences of the *RAD51B* gene. *RAD51B* expression (1.8 kb mRNA isoforms) is ubiquitous, more abundant, and, consistent with its proposed role in DNA repair, radiation-inducible and most prominent in tissues active in recombination. Our results therefore clearly indicate the existence of as yet unknown, uterine splice variants of *RAD51B*.

### Structural characterization of RAD51B.

The established exon/intron distribution revealed that *RAD51B* is composed of 13 exons, the last three of which serve as alternative last exons. Subsequent physical mapping of these exons revealed that *RAD51B* has a genomic size of approximately 0.9 Mb, almost completely encompassing ULCR14 (Fig. 1), with all characterized breakpoints mapping upstream of exon 11^{c} (Fig. 2, arrows in central box). Furthermore, internet-mediated (protein) motif-searching (at http://coot.emblheidelberg.de/SMART/) revealed that RAD51B splice variants containing the unique stretch of 80 amino acids encoded by exon 11^{c} contain a putative carboxyl-terminal transmembrane segment not present in the two previously published splice-variants of *RAD51B*.

### 3' RACE analysis and RT-PCR.

Using 3'-RACE and RT-PCR analysis, we evaluated the existence of t(12;14) translocation-induced hybrid transcripts in uterine leiomyomas. Our results (schematically represented in Fig. 2) revealed that the predicted reading frames of the *RAD51B/HMGIC* transcripts code for variant truncations of the RAD51B protein, all lacking the 80 amino acid domain and containing variable carboxyl-terminal ends, encoded by frameshifted *HMGIC* sequences. The largest subgroup of *RAD51B/HMGIC* hybrids is expected to resemble the fusion transcript in LM-30.1, because the chromosome 12 breakpoints have been reported to frequently map upstream of *HMGIC* , thereby excluding the existence of reciprocal *HMGIC/RAD51B* transcripts. Anyhow, such reciprocal fusion transcripts were detected only in a few cases (Fig. 2), excluding them from a pathogenetically critical role. Expression in the tumors of wild-type *RAD51B* (containing exon 11^{b}) and *HMGIC* transcripts was demonstrated by RT-PCR. In addition expression of exon 11^{a} and exon 11^{c} variants is detected in primary tumors. Exon 11^{c} is also expressed in primary tumors but not in tumor cell lines, while exon 11^{a} and exon 11^{b} expression occurs in both, so in primary tumor and tumor cell lines as well.

### LEGENDS TO THE FIGURES

### Figure 1.

Genomic organization of the *RAD51B* gene on chromosome 14 and expression profiling of novel splice variants.

*Right panel*: Genetic, radiation, and physical (relative to marker AFMA116XD5) maps encompassing the chromosome 14 breakpoint region of uterine leiomyomas (ULCR14), including the relative mapping positions and transcriptional orientations of the *RAD51B* gene as well as two other known genes, i.e. *ACTN1*, encoding a cytoskeleton isoform of a actinin, and *EIF*2, encoding eukaryotic translation initiation factor 2α. The relative positions of anonymous ETSs (black bars) and STSs are also given. The exon/intron distribution of *RAD51B* is also given schematically, with indication of the encoded amino acid residues next to the corresponding exons. The alternatively spliced terminal coding exons are exons 11a-c and they contain different coding sequences.

*Left panel*: Northern blot (Clontech Laboratories, Inc) analysis revealed low level (blot exposure was 14 days) expression of three related transcripts in specimens from uterus (with and without endometrium) but none in a variety of human fetal and adult tissue specimens and cell lines. These transcripts were shown in further studies to represent alternative splice variants of *RAD51B*. It should be noted, furthermore, that thusfar Northern blot level expression of exon 11c has been detected only in primary uterine tissue, indicative for its tissue-restricted expression. Only part of the Northern blot results is shown. Included in these studies were furthermore: i) human adult tissue, including brain, colon (mucosal lining), kidney, liver, lung, pancreas, peripheral blood leukocytes, placenta, stomach, spleen, and thymus; ii) human fetal tissue, including brain, kidney, liver, and lung; iii) cell lines, including A549 (lung carcinoma), G361 (melanoma), HeLa, HL60 (promyelocytic leukemia), K562 (CML), MOLT-4 (lymphoblastic leukemia), and Raji (Burkitt's lymphoma), SW480 (colorectal adenocarcinoma). Molecular weight markers are indicated.

### Figure 2.

### Schematic overview.

Expression of hybrid transcripts in uterine leiomyoma resulting from t(12;14)(q15;q23-24)-induced fusions between *RAD51B* and *HMGIC*.
RT-PCR and 3'-RACE analyses were performed on cytogenetically well-characterized uterine leiomyomas. As an illustration, only the results of four representive and fully characterized cases (LM-30.1, LM-65, LM-100, and LM-608) are presented. Primer sets used were designed on the basis of newly identified as well as known *RAD51B*, and *HMGIC* sequences. Experimental conditions were as described before.
**A**: *HMGIC/RAD51B* hybrid transcript (with numbered exons) and corresponding deduced fusion protein, as detected in cell lines of some of the uterine leiomyomas, as indicated. The three DNA binding domains encoded by exons 1-3 of *HMGIC* are marked.
**B**: In the central box, wild-type mRNA's (with numbered exons) and deduced proteins of the developmentally regulated *HMGIC* gene and the alternatively spliced *RAD51B* are depicted. The two highly conserved nucleotide binding Walker domains in RAD51B are marked (triangles) and the deduced number of amino acids encoded by the three alternative terminal coding exons are indicated. Corresponding chromosome 12 and 14 breakpoints are indicated by labelled arrows.
**C**: *RAD51B/HMGIC* hybrid transcripts and deduced fusion proteins, as detected in cell lines of the indicated uterine leiomyomas. The green bars correspond to amino acid stretches encoded by frameshifted *HMGIC* sequences. Note that in cases similar to LM-30.1, in which the chromosome 12 breakpoints occur upstream of exon 1 of *HMGIC*, no reciprocal *HMGIC/RAD51B* transcripts are formed.

Sequence Listing

SEQ ID.NO.1

GTTGTTAGAACTGTGGCCAGAGTCAGCCCCAGGTCAGAATGCTGGTCAGCA
GCAGACTCACATCCCAGCCCTACCTTCCTGATACCTGATGTGGTGCTCAG
AAAAACAAGGAATCCCAGGCTGTTACCCAGTGTCTCCATGTGCCCTCCAC
AGATGCACTCAACTGTCCTCTCTTCTGCTCTTCCTCCTACAAACTGGGCA
ACTGGAATCAGAACAGAGACTTTGGATGAGCATCCAGTGTAACCCAGCCT
AGTTTTTCACCACGACTTCCAGATCTACATTTAACATCCGACAGCAACTA
TAATTCTACTTTCTGAACCTCCAGGGAAGTTGCTTCCTTGTCCCTTTCAG
CAAAGGATGTCATGGTTTTACAATGTCTCCAGTGAAAAAGTTCTTCTTCT
CTACATCTTCCTCCCACTCCTACAACCCTTGATCCCCACGTGAGATCTCA
TTTCCTCCAGCTGGGTCTTCTGCAGAGTGATTAGAGCTTCTGCCCTCCTT
GCAGCATCTCTATTGTAAAAGGATTGCTGAAGCTTATTAAGCCTTGCAGC
TCCTAGGAGGAAAGGTACCATATAGAAATAAAACAAATCAACAAATTAGT
GTGTTTTGTAGAAGTTGAATAGGACTCCACCTATCTGCATGAGTGTGCAC
ACTGTGCAGTCGCACAGGCAGTCTCAAATGGGAATTTGGAAAAAGCTAAG
CTTTTTCACTTGAGAAAGGTTTACATATTCTTTGCACCTGGGCACACACA
GCACAGGCTGTTCTCAGGACTCCCACCAGATTAGGCCTGGGACATGACAG
AGCCTGTCAGGTCCACGTTTGCTTTCCAGTCTCCTTGCTTCCAGTCTAGC
TGTGGCCTGACTCCAAGTCCAGTGTAAGATTTTTTTTTTCAGCAATAAG
GAATCGGTTCATTCACCTGTACGAAAACCAGTGGGAGGATCATGCATAAG
GCTCATATCCTTGCTGTGAATTTCAAAGGACTGGCTAAGTAAATAAAATT
ATCATGCCAA

SEQ.ID.NO.2

VVRTVARVSPRSECWSAADSHPSPTFLIPDVVLRKTRNPRLLPSVSMCPPQ
MHSTVLSSALPPTNWATGIRTETLDEHPV.

TABLE 1

Intron/Exon Junction Sequences of the human RAD51B gene

Exon | 3' Splice site^a | 5' Splice Site^a
1^b | | GCA GAC CCG gtactggaaa
2 | ggatctggag GC ATG GGT | ACC TGT CAG gtaaatttta
| Met Gly | Thr Cys Gln28
3 | gtttcttgag GAC TTT TTA | ATG CAA ACG gtatatttat
| Asp Phe Leu | Met Gln Thr66
4 | tcttgtttag GCT TAT GGG | CTC ACA GAG gtaaaggaaa
| Ala Tyr Gly | Leu Thr Glu105
5 | tttwttttag ATT ACA GGT | GCT GAA AG gtatgagatw
| Ile Thr Gly | Ala Glu Ar 151
6 | tcacccacag A CTG GTT | CTA CAA AG gtatgctgct
| g Leu Val | Leu Gln Ar 191
7 | tcttttatag G ATT GAA | TCA ATC CCA gtragtkttt
| g Ile Glu | Ser Ile Pro252
8 | ctgttcacag GTT ATC TTG | TCT GAA G gtaaggaatc
| Val Ile Leu | Ser Glu G 285
9 | ttcatttcag GC ACT TCT | AGA AGA CAG gtgggtgctt
| ly Thr Ser | Arg Arg Gln319
10 | ttatgtgcag ATT CTT ATT | CTT CAA G gtaagatcct
| Ile Leu Ile | Leu Gln G 346
| | A 346
| | V 346
11-a | tttcctttag GC CAA GAG | (GTC TAC CAG→ (A)n)
| ly Gln Glu |
11-b | cttcttgcag CC TAT GGA | (TAT AGT GTC→ (A)n)
| la Tyr Gly |
11-c1 | catttcacag TT GTT AGA | (TAT CAT GCC→ (A)n)
| al Val Arg |
11-c2 | catttcacag TT GTT AGA | (nd)
| al Val Arg |
11-c3 | catttcacag TT GTT AGA | (nd)
| al Val Arg |

^a Exon and intron sequences are shown in upper- and lowercase letters, respectively. The consensus dinucleotides gt and ag at the intron borders are underlined.
^b Nucleotides 1-12 (as present only in Genbank accession number U92074) could not be confirmed and were considered to be cloning-artifacts. Exon 1 (our nomenclature) represents an untranslated exon.

nd: not determined

## Claims

1. A DNA sequence partially encoding in adult tissue for an uterus specific protein mapping on human chromosome 14, more specifically on human chromosome 14q23-24 or encoding an immunologically active and/or functional fragment of such a protein, selected from the group consisting of:
(a) DNA sequences comprising a nucleotide sequence encoding a protein comprising the amino acid sequence as given in SEQ ID NO: 2;
(b) DNA sequences comprising a nucleotide sequence as given in SEQ ID NO: 1;
(c) nucleotide sequences hybridizing with one or both of the strands of a DNA sequence as defined in (a) or (b) and encoding an amino acid sequence which is at least 80% identical to the amino acid sequence encoded by the DNA sequence of (a) or (b);
(d) DNA sequences, the nucleotide sequence of which is degenerated as a result of the genetic code to a nucleotide sequence of a DNA sequence as defined in any one of (a) to (c); and
(e) DNA sequences encoding a fragment of a protein encoded by a DNA sequence of any one of (a) to (d).

2. A nucleic acid molecule of at least 15 nucleotides in length designed to hybridize specifically with a DNA sequence of claim 1 or with a complementary strand thereof.

3. A vector comprising a DNA sequence of claim 1 or comprising a nucleic acid molecule of claim 2.

4. The vector of claim 3 which is an expression vector wherein the DNA sequence is operably linked to one or more control sequences allowing the expression in host cells.

5. A host cell containing a vector of claim 3 or 4 or comprising a DNA sequence of claim 1 or a nucleic acid molecule of claim 2.

6. A method for the production of an uterus specific protein or an immunologically active or functional fragment thereof comprising culturing a host cell of claim 5 under conditions allowing the expression of the protein and recovering the produced protein from the culture or from the host cells.

7. An uterus specific protein comprising the amino acid sequence as given in SEQ.ID.NO.2 or an immunologically active or functional fragment thereof encodable by a DNA sequence of claim 1 or obtainable by the method of claim 6.

8. An antibody specifically recognizing the protein of claim 7 or a fragment or epitope thereof.

9. A method for identifying and obtaining an activator or inhibitor of one or more uterus specific proteins comprising the steps of:
(a) combining a compound to be screened with a reaction mixture containing the protein of claim 7 and a system capable of interacting with the protein under suitable conditions;
(b) maintaining said reaction mixture in the presence of the compound or a sample comprising a plurality of compounds under conditions which permit interaction of the protein with said system;
(c) identifying or verifying a sample and compound, respectively, which leads to suppression or activation of said system.

10. A compound obtained or identified by the method of claim 9 which is an activator or inhibitor of uterus specific proteins or which is an activator or inhibitor of a protein activated or inhibited by or interacting with said protein.

11. A diagnostic composition comprising a DNA sequence of claim 1, a vector of claim 3 or 4, a protein of claim 7, an antibody of claim 8, or the compound of claim 10, and optionally suitable means for detection.

12. Use of a DNA sequence of claim 1, a vector of claim 3 or 4, a protein of claim 7, an antibody of claim 8, or the compound of claim 10 for the preparation of a medicament to treat patients suffering from uterine leiomyoma.

13. Pharmaceutical composition comprising one or more of the compounds mentioned in any of the forgoing claims.

14. Animal model for the assessment of the screening for compounds in the treatment of diseases or disorders involving cells with a proliferative capacity such as uterus leiomyomas.

15. Animal model with a genetically modified *RAD51B* gene or any of the genes encoding proteins of claim 7 for the assessment of the screening for compounds in the treatment of diseases or disorders involving cells with a proliferative capacity such as uterus leiomyomas.

16. Transgenic mouse harbouring at least SEQ ID NO.1 or the coding part thereof in its genome.
